# EUROPEAN PATENT SPECIFICATION

(11) **EP 3 691 537 B1**
(45) Date of publication and mention of the grant of the patent: **20.01.2021**
(21) Application number: 19730800.0
(22) Date of filing: 19.06.2019
(51) Int. Cl.: A61B 8/08

(54) **ULTRASOUND ASSISTANCE DEVICE AND MEDICAL SYSTEM**
ULTRASCHALLUNTERSTÜTZUNGSVORRICHTUNG UND MEDIZINISCHES SYSTEM
DISPOSITIF D'ASSISTANCE ULTRASONORE, SYSTÈME MÉDICAL

(30) Priority: 19.06.2018 US 201862687013 P; 10.07.2018 EP 18182671
(43) Date of publication of application: 12.08.2020
(73) Proprietor: Koninklijke Philips N.V., 5656 AG Eindhoven (NL)
(72) Inventor: KRUECKER, Jochen, 5656 AE Eindhoven (NL); STEHLE, Thomas, Heiko, 5656 AE Eindhoven (NL); WENZEL, Fabian, 5656 AE Eindhoven (NL); SCHULZ, Heinrich, 5656 AE Eindhoven (NL)
(74) Representative: Philips Intellectual Property & Standards
(86) International application number: PCT/EP2019/066127
(87) International publication number: WO 2019/243389

(56) References cited:
- WO-A1-2015/086848
- US-A1- 2011 178 389
- WEI SHAO ET AL: "Deformable registration for integration of MRI/MRSI information in TRUS-guided prostate biopsy", VISUAL COMMUNICATIONS AND IMAGE PROCESSING; 20-1-2004 - 20-1-2004; SAN JOSE,, vol. 5747, no. 1, 1 April 2005 (2005-04-01), pages 1263-1273, XP002370563, DOI: 10.1117/12.594897 ISBN: 978-1-62841-730-2

## Description

### FIELD OF THE INVENTION

The present invention relates to an ultrasound assistance device, an ultrasound device including such ultrasound assistance device, a medical system including the same and a corresponding software product.

### BACKGROUND OF THE INVENTION

Prostate cancer can be diagnosed utilizing Magnetic Resonance Imaging (MRI). For optimal MRI image quality, an endorectal coil (ERC) is commonly used. For confirmation of any suspicious lesions identified on MRI, an ultrasound (US)-guided biopsy is typically provided. Ultrasound alone, however, currently does not show prostate cancer with sufficient accuracy. It is thus desirable to co-register and fuse the MRI and US images in order to direct the US-guided biopsy toward the MRI lesions (e.g. using Philips UroNav, which is a system performing MRI-US fusion imaging for prostate (Philips Invivo, Gainesville, FL)).

Deformation of the prostate introduced by the MRI ERC typically differs from the deformation introduced by the US probe, making accurate MRI-US fusion difficult.

In addition, US prostate auto-segmentation is a challenging task, whereas MRI prostate auto-segmentation is more easily accomplished due to the better MRI image resolution and tissue contrast. Segmentations are often needed to accomplish accurate fusion imaging. Due to the different deformations between the MRI and US images of the prostate, an existing MRI prostate segmentation typically cannot be used to create or initialize a corresponding segmentation in the US image.

The paper "Prototype Design and Phantom Evaluation of a Device for Co-registered MRI/TRUS Imaging of the Prostate" by Andriy Fedorov et al. (In: Erdt M. et al. (eds) Clinical Image-Based Procedures. Translational Research in Medical Imaging. CLIP 2013. Lecture Notes in Computer Science, vol 8361. Springer, Cham, pages 125-133, 2013) addresses Magnetic Resonance Imaging (MRI) and transrectal Ultrasound (TRUS) which are both used in imaging interventions in men suspected of having and with prostate cancer for diagnosis as well as treatment. Spatially registered MRI/TRUS data is discussed as providing an optimal combination for characterization of prostate tissue and interventional guidance. To provide such spatially aligned data, the authors of the paper propose a device to support co-registered acquisition of MRI and TRUS data while maintaining a stable configuration (shape) of the prostate. Presented is a design and evaluation of a custom sleeve that can be introduced transrectally, and can accommodate both TRUS and endorectal MRI probes. The experiments on a phantom demonstrate that imaging with this sleeve did not compromise differentiation of internal structures and did not affect the quality of the MR acquisition. Reduction of the signal and contrast were however observed and quantified in the TRUS data. Further evaluation and modification of the device necessary for possible patient studies are discussed.

The need for the sleeve being in position means that the MRI and TRUS scanning are done in more or less immediate succession and does not allow for an independent performing of MRI and TRUS scanning, which is contrary to current clinical workflows.

WO 2015/086848 A1 related to an interventional imaging system for imaging a region of interest within a living being during an interventional procedure. Before the interventional procedure a first ultrasound image is generated by using a first ultrasound endoprobe and a further pre-interventional image is generated (namely MRI), while an element having the same outer dimensions as the first ultrasound endoprobe is arranged within the living being. During the interventional procedure a second ultrasound image is generated by using a second ultrasound endoprobe, the second ultrasound image is registered with the first ultrasound image, thereby determining a deformation, and the further pre-interventional image is deformed in accordance with the determined deformation. This leads to a pre-interventional image, which is deformed in accordance with the current structure of the region of interest and which can be used to accurately guide a physician during the interventional procedure.

Similar to the paper discussed above, the approach of WO 2015/086848 A1 does not correspond to current clinical workflows, which would provide for the pre-interventional MRI scan, e.g. in a hospital, followed some time later by the biopsy, e.g. in a urologist's office. A further concern as to the disclosure of WO 2015/086848 A1 is the provision of the additional pre-interventional ultrasound scan, which puts additional stress on the patient.

### SUMMARY OF THE INVENTION

It is an object of the present invention to provide assistance to the use of ultrasound imaging, in particular in contexts like ultrasound imaging for interventional procedures (e.g. biopsy or brachytherapy), allowing for a segmentation of the ultrasound image of improved quality in comparison to unassisted segmentation, while avoiding drawback involved with known approaches as indicated above, in particular while allowing for an integration with existing clinical workflows, while maintaining a degree of freedom in relation to the obtainment of the additional data used for the assistance and while avoiding additional procedure to be carried out on the patient.

In a first aspect of the present invention an ultrasound assistance device is presented, the ultrasound assistance device comprising an obtaining means for obtaining a segmentation of first image data of a region of interest within a living being, the first image data being acquired by means of a probe using a modality different from ultrasound, and for obtaining deformation information indicative of a first deformation caused by the probe to the living being upon acquiring the first image data, an adjustment means for determining and outputting adjustment data for an adjustable ultrasound probe such that upon acquiring ultrasound image data by means of the ultrasound probe a second deformation is provided to the living body which corresponds to the first deformation, and a segmentation means for segmenting acquired ultrasound image, using the segmentation of the first image data for initialization.

Consideration underlying the present invention include the following:
The different deformations occurring between conventionally obtained prostate MRI images and conventionally obtained US images make MRI-US fusion imaging challenging and limit the fusion accuracy, potentially to the point that suspicious lesions identified in MRI cannot be sampled correctly with, for example, US-guided biopsy. In addition, the different deformations make it difficult to use MRI prostate segmentations, which can be automatically generated, e.g. with the Philips DynaCad software, for initialization, automation or improvement of ultrasound segmentations, which are difficult to generate automatically. This leads to the need for additional manual steps for creating ultrasound segmentations, slows down the workflow, and potentially limits the accuracy of ultrasound segmentations.

The present inventors aimed for an approach in which the form factor and imaging geometry of an ultrasound probe is designed to mimic the form factor of, for example, an MRI ERC as the device for obtaining the other modality, thus creating similar deformations of the prostate during MRI and US imaging. When fusing the images, the MRI prostate segmentation can be used to initialize the US segmentation, thus improving the workflow and the accuracy of the fusion imaging process.

According to the present invention, a segmentation of first image data (e.g. MRI data) and deformation information is obtained, while the deformation information may be obtained explicitly or implicitly (e.g. from general information of the equipment used and/or from the first image data / the segmentation of the first image data). Such deformation information is used for adjustment (e.g. automatic adjustment or guided adjustment including feedback to a user) for an adjustable ultrasound probe, while the ultrasound image acquired by such adjusted ultrasound probe is then segmented using the segmentation of the first image data.

Obtaining ultrasound images with an ultrasound probe that has (almost) the same width (form factor) as the corresponding MR coil has several advantages. In particular, it deforms tissue in the same way as the MR coil, thus leading to optimally matching image features in the MR and US images after either a simple rigid registration, or an elastic registration with a small number of degrees of freedom (e.g. N<10).

It may be noted that the present invention does not imply or require that exactly matching anatomical appearances are provided, which typically is difficult to completely achieve due to differences in, for example, (i) bladder filling, (ii) variations in rectal shape, (iii) variations in folds close to the rectal wall that are caused by different local deformations of the balloon. Therefore, it should be noted that the present invention does not require to make anatomical appearance between US and MRI be identical, but nevertheless allows to significantly improve their similarity. The present invention targets the similarity of the appearance of the prostate between the two modalities, to which the examples of residual apparent difference given above are considered negligible by the inventors and are indeed probably negligible in practice.
In a preferred embodiment, the obtaining means is arranged to receive the first image data and to perform a segmentation processing on the first image data for obtaining the segmentation of the first image data.

While it is possible to provide that segmentation of the first image data is inputted to the ultrasound assistance device (e.g. due to being provided already directly in connection with the acquiring of the first image data itself and after a separate processing of the first image data), the obtaining means may also (in addition or in alternative) be arranged for providing the segmentation based on inputted first image data.

In a preferred embodiment, the obtaining means is arranged to process the segmentation and/or the first image data for obtaining the deformation information.

The deformation information may be provided (in addition or in alternative to being provided explicitly) to the ultrasound assistance device in implicit form, i.e. that the deformation information is derived from the provided data otherwise. Specifically, it is possible to obtain the dimension of the probe causing the deformation from the image data and/or the segmentation.

In the exemplary case of a MR coil being used in the acquiring of the first image data, the MR coil width may be extracted automatically from the MR image, e.g. by fitting a circle of variable radius to the dark, fluid-filled area (i.e. liquid or gas-filled) proximal to the prostate in the MR image.

Similarly, the effected ultrasound probe width (incl. balloon, for example) can be extracted from the ultrasound image by fitting a circle to the dark, fluid-filled area proximal to the prostate in the ultrasound image. Alternatively, the effected ultrasound probe width may be obtained directly from the design of the probe, or from a sensor measuring the width directly or indirectly (e.g. by measuring the flow of fluid into the balloon surrounding the probe).

In a preferred embodiment, the deformation information includes information indicative of a dimension of the probe used for acquiring the first image data.

In a preferred embodiment, the outputting of the adjustment data includes at least one of outputting an adjustment value to a user of the ultrasound assistance device, determining a current adjustment of the adjustable ultrasound probe and indicating a difference between the current adjustment and an adjustment according to the determined adjustment data, and controlling the adjustable ultrasound probe according to the adjustment data.

The adjustment data may be provided to the user (i.e. indicated or displayed), so to allow the user to provide, by appropriate means, the adjustment of the ultrasound probe, e.g. manually.

Further, rather (or in addition to) outputting an indication of the absolute adjustment, the user may be provided with an indication about a deviation between a current adjustment and the determined adjustment conforming with the deformation information.

Yet further, it is also foreseen that the adjustment of the ultrasound probe is done automatically by the ultrasound assistance device.

For example, the adjustable ultrasound probe may be provided with an ultrasound compatible balloon, wherein the width of which can be displayed to the user, based on the measured fluid injection volume and width-volume calibration, or based on sensors attached to the surface of the balloon (e.g. resistive, ultrasonic, or electromagnetic).

The fluid filling can be done automatically using a software-controlled pump injecting fluid until the desired width w is reached. Alternatively the fluid filling can be manual, with a software providing feedback to the user about the current and/or desired width of the balloon on a display.

In a preferred embodiment, the ultrasound assistance device further comprises a registration means for registering an image based on the first image data and the ultrasound image and for outputting a fusioned image based on the registration.

The fusion image allows the practitioner to grasp the combined information from the ultrasound imaging and the previously obtained first image data more readily in comparison to, for example, a parallel display of the images separately.

In a preferred embodiment, the first image data is acquired by means of Magnetic Resonance Imaging and the probe includes a MRI coil.

It is preferred that the first image modality is or includes MRI, while nevertheless other modalities (e.g. CT) are also possible.

In a preferred embodiment, the region of interest is a prostrate, the probe is an endorectal probe and the ultrasound probe is a transrectal ultrasound.

It was found that the present invention is particularly beneficial for use in the context of imaging regarding the prostrate, wherein ultrasound imaging is employed for guiding biopsy procedures and/or brachytherapy procedure, while, nevertheless, other applications are also possible.

The present invention may be used, for example, for Philips UroNav or PercuNav system, and may be used for other applications employing multi-modality registration involving images acquired with endocavity probes or with probes that affect the geometry or deformation of the organ being imaged.

In a preferred embodiment, an ultrasound device is provided comprising the ultrasound assistance device according to the present invention, and an adjustable ultrasound probe.

In a preferred embodiment, the adjustable ultrasound probe is provided with an inflatable balloon, which is fluid filled or fluid fillable, and/or one or more controllable mechanical elements acting on a flexible jacket.

In a further embodiment, the adjustable ultrasound probe may consist of at least two parts removably connected to each other, a fixed top part containing the ultrasound transducer array facing the prostate, and an interchangeable bottom part on the opposite side, which serves to increase the overall probe size to fit the deformation induced by the first imaging modality. The bottom part can be chosen from a set of pre-fabricated parts of different sizes, or can be 3D-printed based on the information obtained from the first imaging modality.

In yet a further aspect of the present invention a software product for ultrasound assistance is presented, the software product comprising program code means for causing an ultrasound assistance device according to the present invention to carry out the steps of the method according to the present invention when the software product is run on the ultrasound assistance device.

It shall be understood that the ultrasound assistance device of claim 1 and the software product of claim 12 have similar and/or identical preferred embodiments, in particular, as defined in the dependent claims. It shall be understood that a preferred embodiment of the invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 schematically shows a medical system in accordance with an embodiment of the invention,
Fig. 2 schematically shows an MR imaging coil and two adjustable ultrasound probes in accordance with embodiments of the invention,
Fig. 3 shows an illustration of image-based determination of an endorectal coil width, and
Fig. 4 shows a flow diagram illustrating a method according to an embodiment of the present invention.

### DETAILED DESCRIPTION OF EMBODIMENTS

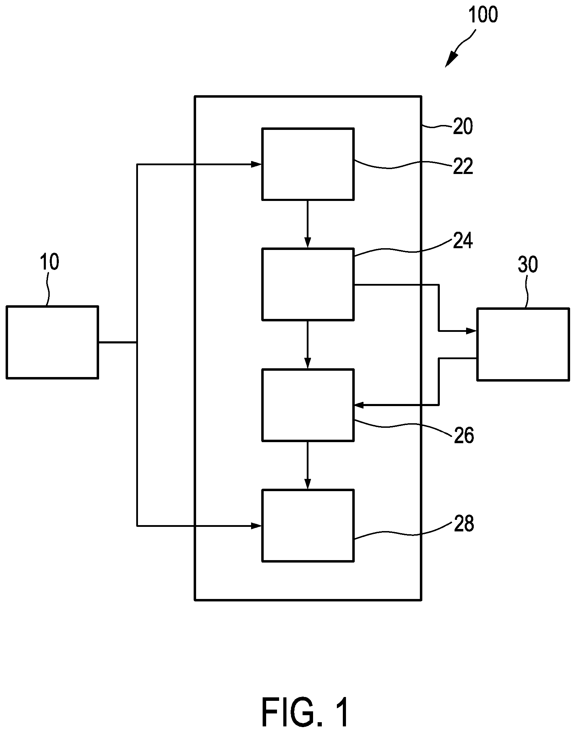

Fig. 1 schematically shows a medical system in accordance with an embodiment of the invention.

The medical system 100 according to the present embodiment includes a MR imaging system 10, an ultrasound device including an ultrasound assistance device 20 and an adjustable ultrasound probe 30.

The ultrasound assistance device 20 includes an obtaining unit 22, an adjustment unit 24, a segmentation unit 26 and a registering unit 28.

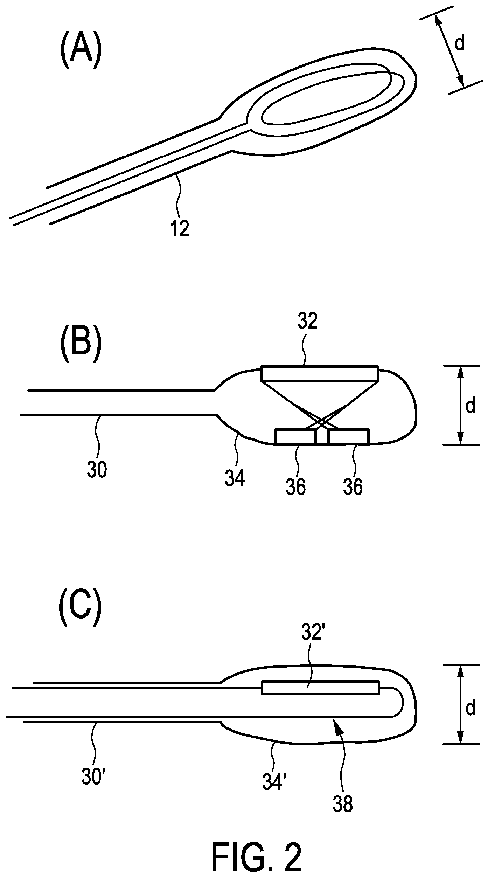

The MR imaging system 10 is configured to image a prostate with an endorectal coil (ERC) (see Fig. 2 a)).
The ultrasound probe 30 is adjustable, such that it can assume an imaging geometry and form factor that allows prostate ultrasound image acquisition with basically the same deformations to the prostate as during MRI imaging.

A workflow implemented by the medical system 100 includes that the MR imaging system 10 images the prostate using MRI with the ERC. Further, the prostate is segmented by the MR imaging system 10 in the MR image.

Later, the ultrasound device is arranged to image the prostate with the ultrasound probe 30 that has - due to the adjustment - basically the same form factor as the ERC.

The ultrasound assistance device 20 and more specifically the segmentation unit 26 further maps the MRI segmentation on the ultrasound image to initialize ultrasound segmentation and automatically segments the prostate in the US image, based on the MRI initialization.

Furthermore, the ultrasound assistance device 20 and more specifically the registering unit 28 registers the MR and ultrasound images based on the segmentations and uses the registered MRI/US images to perform fusion imaging or fusion biopsy guidance.

Fig. 2 schematically shows an MR imaging coil and two adjustable ultrasound probe in accordance with embodiments of the invention.

Fig. 2 a) illustrates a conventionally known endorectal probe 12 including an MR coil. The probe has a diameter d which is larger than diameters of typically known ultrasound probes for prostate applications. As such endorectal MR probe is well known to the skilled person, no further discussion is provided.

Fig. 2 b) illustrates an adjustable ultrasound probe 30 in accordance with an embodiment of the invention. The probe 30 includes an ultrasound imaging array 32 and is further provided, on the side opposite to the imaging array 32 (i.e. facing away from the imaging direction), with a flexible jacket 34, which is deformed by wings 36 of the probe 30, wherein the diameter of the probe 30 may thus be adjusted.

Fig. 2 c)) illustrates another adjustable ultrasound probe 30' in accordance with an embodiment of the invention. The adjustable probe 30' also includes an ultrasound imaging array 32', while, however, the distal portion (i.e. the illustrated portion) is enclosed by an adjustable balloon 34' filled with fluid 38. By adjusting the amount of fluid 38 provided inside the balloon 34', the diameter thereof may be adjusted. As such, such adjustable ultrasound probe including a balloon is known, and therefore no further explanation thereof might be needed.

According to the present invention, the adjustable ultrasound probe is provided such that - in operation - the active tip containing the imaging array has the same diameter d as the endorectal MR imaging coil used during MR imaging. During ultrasound scanning, the contact of the US imaging probe with the tissue will thus give rise to the same/similar tissue deformation as during MR imaging. In order to accommodate MR images obtained with coils of variable width d, an ultrasound probe can, for example, be fitted with a fluid-filled or fluid-fillable balloon that can be inflated to the same width d as the MR imaging coil.

The balloon width can be calibrated with respect to the fluid fill volume, giving the width d as a function of the injected fluid volume. Based on the MR coil width used, the user can inject the desired fluid volume to reach width d.

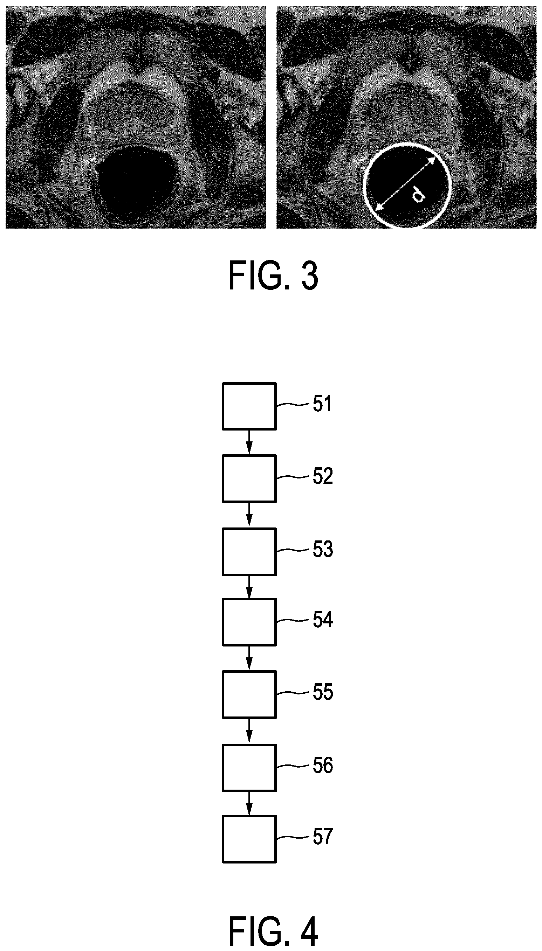

Fig. 3 shows an illustration of image-based determination of an endorectal coil width. From Fig. 3, one can see that it is possible to determine the diameter of the probe used by fitting a circle to the dark area resulting from the probe.

Fig. 4 shows a flow diagram illustrating a method according to an embodiment of the present invention.

The method starts in this case with an obtaining (51) of a segmentation of first image data of a region of interest within a living being, wherein the first image data was previously acquired by means of an MRI probe (endorectal coil).

Further, the method includes obtaining (52) deformation information indicative of a first deformation caused by the probe to the living being upon acquiring the first image data.

Based on the deformation information, the method further includes determining (53) adjustment data for an adjustable ultrasound probe such that upon acquiring ultrasound image data by means of the ultrasound probe a second deformation is provided to the living body which corresponds to the first deformation.

The adjustment data is then used for controlling (54) the adjustable ultrasound probe.

An ultrasound image acquired using the adjusted ultrasound probe is then segmented (55), using the segmentation of the first image data for initialization.

Furthermore, a fusioned image is generated or registered (56) based on the first image data and the ultrasound image and the fusioned image is then outputted (57).

For accurate fusion imaging and other clinical tasks such as determination of prostate volume it is desirable to segment a 3-dimension ultrasound (3DUS) image of the prostate. The shape and thus the segmentation of the prostate depends on the deformation on the prostate introduced by the ultrasound probe. A workflow including an obtaining of a 3D MRI can with an endorectal coil (ERC), an obtaining of a prostrate segmentation in the 3D MRI, an obtaining of a 3DUS scan with an ultrasound probe that is matched in size to the ERC, a mapping of the MRI segmentation on the 3DUS so to initialize automatic 3DUS segmentation and an adjusting of the segmentation to fit the prostate contours in the 3DUS can be used to extract the ultrasound segmentation accurately and robustly, based on a segmentation already performed on the prior MRI volume.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single processor, device or other unit may fulfill the functions of several items recited in the claims. The mere fact that certain measures are recited in mutually different dependent claims does not indicate that a combination of these measures cannot be used to advantage.

Operations like segmenting, calculating, determining, outputting, processing and the like can be implemented as program code means of a computer program and/or as dedicated hardware.

A computer program may be stored and/or distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. An ultrasound assistance device (20), comprising:
- an obtaining means (22) for obtaining a segmentation of first image data of a region of interest within a living being, the first image data being acquired by means of a probe (12) using a modality different from ultrasound, and for obtaining deformation information indicative of a first deformation caused by the probe (12) to the living being upon acquiring the first image data,
- an adjustment means (24) for determining and outputting adjustment data for an adjustable ultrasound probe (30, 30') such that upon acquiring ultrasound image data by means of the ultrasound probe (30, 30') a second deformation is provided to the living body which corresponds to the first deformation, and
- a segmentation means (26) for segmenting acquired ultrasound image, using the segmentation of the first image data for initialization.

2. The ultrasound assistance device (20) according to claim 1,
wherein the obtaining means (22) is arranged to receive the first image data and to perform a segmentation processing on the first image data for obtaining the segmentation of the first image data.

3. The ultrasound assistance device (20) according to claim 1,
wherein the obtaining means (22) is arranged to process the segmentation and/or the first image data for obtaining the deformation information.

4. The ultrasound assistance device (20) according to claim 1,
wherein the deformation information includes information indicative of a dimension of the probe (12) used for acquiring the first image data.

5. The ultrasound assistance device (20) according to claim 1,
wherein the outputting of the adjustment data includes at least one of outputting an adjustment value to a user of the ultrasound assistance device,
determining a current adjustment of the adjustable ultrasound probe and indicating a difference between the current adjustment and an adjustment according to the determined adjustment data, and
controlling the adjustable ultrasound probe (30, 30') according to the adjustment data.

6. The ultrasound assistance device (20) according to claim 1, further comprising:
a registration means (28) for registering an image based on the first image data and the ultrasound image and for outputting a fusioned image based on the registration.

7. The ultrasound assistance device (20) according to claim 1,
wherein the first image data is acquired by means of Magnetic Resonance Imaging and the probe (12) includes a MRI coil.

8. The ultrasound assistance device (20) according to claim 1,
wherein the region of interest is a prostrate, the probe is an endorectal probe (12) and the ultrasound probe is a transrectal ultrasound probe (30, 30').

9. An ultrasound device, comprising:
- the ultrasound assistance device (20) according to claim 1, and
- an adjustable ultrasound probe (30, 30').

10. The ultrasound device according to claim 9,
wherein the adjustable ultrasound probe (30, 30') is provided with
- an inflatable balloon (34'), which is fluid filled or fluid fillable, and/or
- one or more controllable mechanical elements (36) acting on a flexible jacket (34).

11. A medical system (100), comprising:
- the ultrasound device according to claim 1, and
- a Magnetic Resonance Imaging device (10),
wherein the Magnetic Resonance Imaging device (10) comprises a probe (12) including an MRI coil and is arranged to provide Magnetic Resonance Imaging data to the obtaining means of the ultrasound assistance device (20).

12. A software product for ultrasound assistance, the software product comprising program code means for causing an ultrasound assistance device (20) according to claim 1 to carry out the steps of the method:
- obtaining (51) a segmentation of first image data of a region of interest within a living being, the first image data being acquired by means of a probe (12) using a modality different from ultrasound,
- obtaining (52) deformation information indicative of a first deformation caused by the probe (12) to the living being upon acquiring the first image data,
- determining (53) adjustment data for an adjustable ultrasound probe (30, 30') such that upon acquiring ultrasound image data by means of the ultrasound probe (30, 30') a second deformation is provided to the living body which corresponds to the first deformation, outputting (54) the adjustment data, and
- segmenting (55) acquired ultrasound image, using the segmentation of the first image data for initialization;
when the software product is run on the ultrasound assistance device (20).

## Patentansprüche

1. Ultraschallunterstützungsvorrichtung (20), umfassend:
- ein Erhaltungsmittel (22) zum Erhalten einer Segmentierung von ersten Bilddaten eines interessierenden Bereichs innerhalb eines Lebewesens, wobei die ersten Bilddaten mittels einer Sonde (12) unter Verwendung einer von Ultraschall verschiedenen Modalität erfasst werden, und zum Erhalten von Verformungsinformationen, die eine erste Verformung anzeigen, die durch die Sonde (12) für das Lebewesen beim Erfassen der ersten Bilddaten verursacht wird,
- ein Einstellmittel (24) zum Bestimmen und Ausgeben von Einstelldaten für eine einstellbare Ultraschallsonde (30, 30'), so dass beim Erfassen von Ultraschallbilddaten mittels der Ultraschallsonde (30, 30') dem lebenden Körper eine zweite Verformung bereitgestellt wird, die der ersten Verformung entspricht, und
- ein Segmentierungsmittel (26) zum Segmentieren des erfassten Ultraschallbildes unter Verwendung der Segmentierung der ersten Bilddaten zur Initialisierung.

2. Ultraschallunterstützungsvorrichtung (20) nach Anspruch 1,
wobei das Erhaltungsmittel (22) angeordnet ist, um die ersten Bilddaten zu empfangen, und eine Segmentierungsverarbeitung an den ersten Bilddaten durchzuführen, um die Segmentierung der ersten Bilddaten zu erhalten.

3. Ultraschallunterstützungsvorrichtung (20) nach Anspruch 1,
wobei das Erhaltungsmittel (22) angeordnet ist, um die Segmentierung und/oder die ersten Bilddaten zum Erhalten der Verformungsinformationen zu verarbeiten.

4. Ultraschallunterstützungsvorrichtung (20) nach Anspruch 1,
wobei die Verformungsinformation Informationen enthält, die eine Abmessung der Sonde (12) angeben, die zum Erfassen der ersten Bilddaten verwendet wird.

5. Ultraschallunterstützungsvorrichtung (20) nach Anspruch 1,
wobei die Ausgabe der Einstelldaten mindestens eines der Folgenden umfasst
Ausgabe eines Einstellwerts an einen Benutzer der Ultraschallunterstützungsvorrichtung,
Bestimmen einer Stromeinstellung der einstellbaren Ultraschallsonde und Anzeigen einer Differenz zwischen der Stromeinstellung und einer Einstellung gemäß den ermittelten Einstelldaten, und
Steuerung der einstellbaren Ultraschallsonde (30, 30') gemäß den Einstelldaten.

6. Ultraschallunterstützungsvorrichtung (20) nach Anspruch 1, ferner umfassend:
ein Anpassungsmittel (28) zum Anpassen eines Bildes basierend auf den ersten Bilddaten und dem Ultraschallbild und zum Ausgeben eines fusionierten Bildes basierend auf der Anpassung.

7. Ultraschallunterstützungsvorrichtung (20) nach Anspruch 1,
wobei die ersten Bilddaten mittels Magnetresonanzbildgebung erfasst werden, und die Sonde (12) eine MRI-Spule enthält.

8. Ultraschallunterstützungsvorrichtung (20) nach Anspruch 1,
wobei der interessierende Bereich eine Prostata ist, die Sonde eine endorektale Sonde (12) ist und die Ultraschallsonde eine transrektale Ultraschallsonde (30, 30') ist.

9. Ultraschallvorrichtung, umfassend:
- die Ultraschallunterstützungsvorrichtung (20) nach Anspruch 1, und
- eine einstellbare Ultraschallsonde (30, 30').

10. Ultraschallvorrichtung nach Anspruch 9,
wobei die einstellbare Ultraschallsonde (30, 30') versehen ist, mit
- einen aufblasbaren Ballon (34'), der mit Flüssigkeit gefüllt oder mit Flüssigkeit befüllbar ist, und/oder
- ein oder mehrere steuerbare mechanische Elemente (36), die auf einen flexiblen Mantel (34) wirken.

11. Medizinisches System (100), umfassend:
- die Ultraschallvorrichtung nach Anspruch 1 und
- eine Magnetresonanzbildgebungsvorrichtung (10), wobei die Magnetresonanzbildgebungsvorrichtung (10) eine Sonde (12) mit einer MRI-Spule umfasst und angeordnet ist, um Magnetresonanzbildgebungsdaten an die Erhaltungsmitteln der Ultraschallunterstützungsvorrichtung (20) bereitzustellen.

12. Softwareprodukt zur Ultraschallunterstützung, wobei das Softwareprodukt ein Programmcodemittel umfasst, um eine Ultraschallunterstützungsvorrichtung (20) gemäß Anspruch 1 zu veranlassen, die Schritte des Verfahrens auszuführen:
- Erhalten (51) einer Segmentierung der ersten Bilddaten eines interessierenden Bereichs innerhalb eines Lebewesens, wobei die ersten Bilddaten mittels einer Sonde (12) unter Verwendung einer von Ultraschall verschiedenen Modalität erfasst werden;
- Erhalten von (52) Verformungsinformationen, die eine erste Verformung anzeigen, die durch die Sonde (12) für das Lebewesen beim Erfassen der ersten Bilddaten verursacht wird,
- Bestimmen (53) von Einstelldaten für eine einstellbare Ultraschallsonde (30, 30'), so dass beim Erfassen von Ultraschallbilddaten mittels der Ultraschallsonde (30, 30') dem lebenden Körper eine zweite Verformung bereitgestellt wird, die der ersten Verformung entspricht,
- Ausgabe (54) der Einstelldaten, und
- Segmentieren (55) des erfassten Ultraschallbildes unter Verwendung der Segmentierung der ersten Bilddaten zur Initialisierung;
wenn das Softwareprodukt auf der Ultraschallunterstützungsvorrichtung (20) ausgeführt wird.

## Revendications

1. Dispositif d'assistance ultrasonore (20), comprenant:
- un moyen d'obtention (22) pour obtenir une segmentation de premières données d'image d'une région d'intérêt dans un être vivant, les premières données d'image étant acquises au moyen d'une sonde (12) en utilisant une modalité différente des ultrasons, et pour obtenir des informations de déformation indiquant une première déformation causée par la sonde (12) à l'être vivant lors de l'acquisition des premières données d'image,
- un moyen d'ajustement (24) pour déterminer et sortir des données d'ajustement pour une sonde ultrasonore ajustable (30, 30') de telle sorte que lors de l'acquisition de données d'image ultrasonore au moyen de la sonde ultrasonore (30, 30'), une deuxième déformation est fournie au corps vivant qui correspond à la première déformation, et
- un moyen de segmentation (26) pour segmenter l'image ultrasonore acquise, en utilisant la segmentation des premières données d'image pour l'initialisation.

2. Dispositif d'assistance ultrasonore (20) selon la revendication 1,
dans lequel le moyen d'obtention (22) est agencé pour recevoir les premières données d'image et pour effectuer un traitement de segmentation sur les premières données d'image pour obtenir la segmentation des premières données d'image.

3. Dispositif d'assistance ultrasonore (20) selon la revendication 1,
dans lequel le moyen d'obtention (22) est agencé pour traiter la segmentation et/ou les premières données d'image pour obtenir les informations de déformation.

4. Dispositif d'assistance ultrasonore (20) selon la revendication 1,
dans lequel les informations de déformation comprennent des informations indiquant une dimension de la sonde (12) utilisée pour acquérir les premières données d'image.

5. Dispositif d'assistance ultrasonore (20) selon la revendication 1,
dans lequel la sortie des données d'ajustement comprend au moins un parmi
sortir une valeur d'ajustement à un utilisateur du dispositif d'assistance ultrasonore,
déterminer un ajustement courant de la sonde ultrasonore ajustable et indiquer une différence entre l'ajustement courant et un ajustement selon les données d'ajustement déterminées, et
commander la sonde ultrasonore ajustable (30, 30') selon les données d'ajustement.

6. Dispositif d'assistance ultrasonore (20) selon la revendication 1, comprenant en outre:
un moyen d'alignement (28) pour aligner une image sur la base des premières données d'image et de l'image ultrasonore et pour sortir une image fusionnée sur la base de l'alignement.

7. Dispositif d'assistance ultrasonore (20) selon la revendication 1,
dans lequel les premières données d'image sont acquises au moyen d'une Imagerie par Résonance Magnétique et la sonde (12) comprend une bobine d'IRM.

8. Dispositif d'assistance ultrasonore (20) selon la revendication 1,
dans lequel la région d'intérêt est une prostate, la sonde est une sonde endorectale (12) et la sonde ultrasonore est une sonde ultrasonore transrectale (30, 30').

9. Dispositif ultrasonore, comprenant:
- le dispositif d'assistance ultrasonore (20) selon la revendication 1, et
- une sonde ultrasonore ajustable (30, 30').

10. Dispositif ultrasonore selon la revendication 9, dans lequel la sonde ultrasonore ajustable (30, 30') est pourvue de
- un ballon gonflable (34'), qui est rempli de fluide ou peut être rempli de fluide, et/ou
- un ou plusieurs éléments mécaniques contrôlables (36) agissant sur une chemise souple (34).

11. Système médical (100), comprenant:
- le dispositif ultrasonore selon la revendication 1, et
- un dispositif d'Imagerie par Résonance Magnétique (10),
où le dispositif d'Imagerie par Résonance Magnétique (10) comprend une sonde (12) comprenant une bobine d'IRM et est agencé pour fournir des données d'Imagerie par Résonance Magnétique aux moyens d'obtention du dispositif d'assistance ultrasonore (20) .

12. Logiciel d'assistance ultrasonore, le produit logiciel comprenant des moyens de code de programme pour amener un dispositif d'assistance ultrasonore (20) selon la revendication 1 à exécuter les étapes du procédé:
- obtenir (51) une segmentation de premières données d'image d'une région d'intérêt dans un être vivant, les premières données d'image étant acquises au moyen d'une sonde (12) en utilisant une modalité différente des ultrasons,
- obtenir (52) des informations de déformation indiquant une première déformation causée par la sonde (12) à l'être vivant lors de l'acquisition des premières données d'image,
- déterminer (53) des données d'ajustement pour une sonde ultrasonore ajustable (30, 30') de telle sorte que lors de l'acquisition de données d'image ultrasonore au moyen de la sonde ultrasonore (30, 30') une deuxième déformation est fournie au corps vivant qui correspond à la première déformation,
- sortir (54) les données d'ajustement, et
- segmenter (55) l'image ultrasonore acquise, en utilisant la segmentation des premières données d'image pour l'initialisation;
lorsque le produit logiciel est exécuté sur le dispositif d'assistance ultrasonore (20).
